# FASCICULE DE BREVET EUROPEEN

(11) **EP 1 531 779 B1**
(45) Date de publication et mention de la délivrance du brevet: **06.01.2010**
(21) Numéro de dépôt: 03792448.7
(22) Date de dépôt: 13.08.2003
(51) Int. Cl.: A61K 6/06

(54) **PREPARATION POUR REALISER UN MATERIAU DE RESTAURATION DE SUBSTANCE MINERALISEE, NOTAMMENT DANS LE DOMAINE DENTAIRE**
ZUSAMMEMSETZUNG ZUR HERSTELLUNG VON EINEM MINERALISCHEN RESTAURATIONSMATERIA INSBESONDERE FÜR DEN DENTALBEREICH
PREPARATION FOR PRODUCING A MATERIAL USED TO RESTORE A MINERALISED SUBSTANCE, PARTICULARLY IN THE DENTAL FIELD

(30) Priorité: 23.08.2002 FR 0210539
(43) Date de publication de la demande: 25.05.2005
(73) Titulaire: Septodont ou Specialites Septodont S.A., 94100 St Maur des Fosses (FR)
(72) Inventeur: BERGAYA, Badreddine, F-45590 Saint-Cyr en Val (FR); BOTTERO, Jean-Yves, Domaine de Calas, 13480 Cabries (FR); BOTTERO, Marie-Josée, Domaine de Calas, 13480 Cabries (FR); FRANQUIN, Jean-Claude, F-13008 Marseille (FR); LEBLANC, Dominique, F-75005 Paris (FR); MARIE, olivier, F-91450 Soisy sur Seine (FR); NONAT, André, F-21380 Epagny (FR); SAUVAGET, Cyrille chez Mme Sandrine Sauvaget, 13610 Le Puy Saint Reparade (FR)
(74) Mandataire: Bentz, Jean-Paul
(86) Numéro de dépôt international: PCT/FR2003/002528
(87) Numéro de publication internationale: WO 2004/017929

(56) Documents cités:
- WO-A-01/76534
- DE-A- 19 923 956
- US-A- 5 415 547
- ANDREEVA E P ET AL: "MECHANISM OF EFFECT OF CALCIUM CHLORIDE ON PROCESSES OF DISPERSE STRUCTURE FORMATION, AND CHEMICAL INTERACTION IN THE HYDRATION OF beta DICALCIUM AND TRICALCIUM SILICATES" COLLOID J USSR JUL-AUG 1982, vol. 44, no. 4, juillet 1982 (1982-07), pages 568-573, XP0008017954
- PATENT ABSTRACTS OF JAPAN vol. 0154, no. 03 (C-0875), 15 octobre 1991 (1991-10-15) & JP 3 165773 A (TDK CORP), 17 juillet 1991 (1991-07-17)

## Description

La présente invention concerne une préparation pour réaliser un matériau de restauration de substance minéralisée, notamment dans le domaine dentaire.

Dans le domaine dentaire, la restauration des couronnes vise à pallier, entre autres, la perte de substance liée à la destruction des tissus dentaires par la carie ou à la suite d'un choc.

A ce jour, la reconstitution des dents "délabrées" représente 75 à 80% des traitements dentaires (P. Hescot et al.; 1996 ; Programme International de Recherche de l'Organisation Mondiale de la Santé sur les déterminants et la santé bucco-dentaire ; Association Dentaire Française).

Actuellement, la reconstitution dentaire utilise essentiellement deux types de matériaux de restauration évitant de recourir à l'intervention d'un laboratoire de prothèses.

Le matériau le plus ancien, utilisé depuis le XIX^{ème} siècle, est l'amalgame d'argent.

Très largement répandu, sa mise en place par le praticien est très aisée et sa résistance mécanique n'est plus à démontrer. En outre, la durée de vie moyenne d'une obturation à l'amalgame est estimée à 14 ans.

Toutefois, l'amalgame d'argent présente deux inconvénients majeurs.

Le premier inconvénient est lié à la présence de 40 à 50% de mercure dans sa formulation. Le danger éventuel d'un relargage du mercure dans la salive et dans l'atmosphère lors de la mise en place du matériau, ainsi que dans les eaux usées lors de sa dépose, a conduit à un rejet progressif de l'utilisation de ce type de matériau.

Le deuxième inconvénient est lié à l'aspect métallique inesthétique des obturations réalisées au moyen d'un amalgame d'argent.

Pour pallier la présence de mercure d'une part, et à l'aspect inesthétique des amalgames d'argent d'autre part, un deuxième type de matériau de restauration a été développé. Il s'agit des résines composites.

Les résines composites sont formées par un mélange de résine organique et de charges minérales, le mélange étant spécialement traité par un produit qui assure une liaison entre la résine et les charges minérales, en l'absence totale de mercure.

Destinées originellement au traitement des dents antérieures, car elles répondent à la demande esthétique des patients, elles sont également utilisées pour la reconstitution des dents postérieures.

Toutefois, on constate que les obturations réalisées avec ces résines composites présentent une durée moyenne de vie estimée à 7 ans, soit deux fois moindre que celle des obturations à l'amalgame d'argent.

Cette faible durée moyenne de vie des obturations en résine composite s'explique par le phénomène de contraction de la résine composite qui se produit lors de la réaction de prise des résines composites et qui ne permet plus d'assurer une étanchéité marginale satisfaisante lors de sa réaction de polymérisation, ce qui constitue un problème majeur dans l'utilisation de telles résines.

A ce jour, et malgré de nombreuses tentatives visant à améliorer les composants des résines composites ainsi que les techniques d'utilisation associées, aucune résine composite ne présente d'étanchéité marginale satisfaisante, notamment dans les zones où l'émail est peu ou pas présent.

Aussi, les avantages écologique et esthétique des résines composites se traduisent par une régression en terme de santé publique et d'économie du point de vue du budget de la santé.

Andreeva et al. (« Mechanism of effect of calcium chloride on processes of disperse structure formation, and chemical interaction in the hydration of beta dicalcium and tricalcium silicates», Colloid J. USSR, vol. 44, No 4, Juillet 1982, pages 568-573) décrit la cinétique de la réaction du chlorure de calcium sur les mélanges de silicates tricalciques et dicalciques avec du carbonate de calcium. Toutefois, les propriétés de la composition obtenue par cette réaction ne permettent pas son utilisation comme matériau de restauration d'une substance minéralisée tel que dans le domaine dentaire.

WO 01/76534 décrit une méthode pour obtenir un matériau céramique lié chimiquement, pour une utilisation dans le domaine des ciments de construction mais aussi dans le domaine dentaire, du type CaO-Al₂O₃-(SiO₂) -H₂O.

DE 199 23 956 décrit un matériau céramique de restauration dentaire, à base de silicate tricalcique. Le matériau est sous la forme d'un kit de deux composants, un solide et un liquide qui sont mélangés au moment de l'usage. Toutefois, ce matériau ne présente pas des caractéristiques tout à fait satisfaisantes.

Il y a donc un besoin réel consistant à disposer d'un matériau de restauration pour la reconstitution dentaire, ledit matériau offrant un compromis entre les avantages des amalgames d'argent, notamment en termes de longévité et de résistance mécanique, et ceux des résines composites, à savoir absence de mercure et aspect esthétique de l'obturation.

La présente invention a ainsi pour objectif de pouvoir réaliser un matériau de restauration de substance minéralisée, en particulier pour la restauration dentaire, ce matériau étant apte à résister à des pressions de l'ordre d'au moins 100 Mpa, étant en outre stable dimensionnellement durant sa mise en place et après celle-ci, et ayant enfin une bonne adhésion avec la substance minérale qu'il permet de restaurer.

Un objectif additionnel de l'invention, notamment dans ses applications dentaires, est de pouvoir réaliser un matériau ayant les caractéristiques précédentes et qui de plus doit être biocompatible.

Dans le domaine spécifiquement dentaire, ce matériau doit de surcroît permettre d'obtenir une bonne étanchéité marginale en l'absence de tout phénomène de contraction linéaire pour assurer une durée moyenne de vie semblable à celle des amalgames d'argent, permettre une obturation esthétique, résister aux pressions masticatrices qui sont dans la gamme des valeurs de pression indiquées ci-dessus, et avoir un temps de prise compatible avec la durée de manipulation nécessaire au chirurgien dentiste qui est de 10 à 30 minutes.

Selon l'invention, une préparation permettant de réaliser un tel matériau comprend
- une partie liquide aqueuse,
- une partie solide comprenant au moins un silicate choisi parmi le silicate tricalcique Ca₃SiO₅ et le silicate dicalcique Ca₂SiO₄,
- du chlorure de calcium CaCl₂ et un agent réducteur d'eau, contenus chacun dans au moins l'une des dites parties,
   la partie solide et la partie liquide étant destinées à être mélangées pour obtenir ledit matériau.

On appelle ainsi "partie solide" l'ensemble des phases solides que sont les poudres de chacun de ses constituants, et "partie liquide" la phase liquide aqueuse dans laquelle peuvent être rajoutés à l'eau les autres constituants qui composent alors cette partie ou phase liquide.

L'agent réducteur d'eau permet de limiter l'apport d'eau et donc d'adapter le volume de la phase liquide par rapport à celui de la phase solide, sans que l'hydratation du silicate tricalcique et/ou du silicate dicalcique, qui se produit lors du mélange des phases solide et liquide, ne soit altérée.

L'agent réducteur d'eau présente de manière avantageuse un pouvoir fluidifiant et/ou plastifiant.

De telles propriétés de l'agent réducteur d'eau permettent ainsi de conférer une fluidité ainsi qu'une plasticité au matériau obtenu après mélange de la préparation selon l'invention, rendant le malaxage et la manipulation du matériau plus aisés pour le praticien.

On peut notamment utiliser des plastifiants comme agents réducteurs d'eau, tels que par exemple du polynaphtalènesulfonate (PNS) ou un plastifiant, dit "superplastifiant", à base de polycarboxylate modifié.

Pour la restauration dentaire, la partie solide comprend en outre du carbonate de calcium CaCO₃.

Le carbonate de calcium a un effet accélérateur sur l'hydratation du silicate de calcium, dicalcique ou tricalcique.

En outre, il permet d'augmenter les propriétés de résistance à la compression du matériau obtenu après mélange de la préparation selon l'invention.

De préférence, dans le domaine dentaire, la partie solide comprend entre 70% et 99%, en poids de silicate dicalcique et/ou tricalcique, et entre 1 et 30% en poids de carbonate de calcium CaCO₃, ces pourcentages pondéraux étant donnés en référence à l'ensemble des constituants de la partie solide.

De manière préférée, la partie solide comprend de l'oxyde de zirconium ZᵣO₂.

L'oxyde de zirconium présente un double avantage.

D'une dureté remarquable, il améliore encore les propriétés mécaniques du matériau obtenu à partir de la préparation selon l'invention.

En outre, en augmentant la radio-opacité de ce matériau, il permet au praticien un contrôle radiographique amélioré de la restauration de substance minéralisée effectuée.

De préférence, la proportion d'oxyde de zirconium est comprise entre 0 et 15% en poids de l'ensemble des constituants de cette partie solide.

Selon un mode de réalisation valable pour l'ensemble des applications de la présente invention, c'est la partie liquide qui comprend la CaCl₂.

La teneur en CaCl₂ peut par exemple être comprise entre 1 et 35%, et de préférence entre 9 et 25%, en poids par rapport au volume total de cette partie liquide.

Dans un autre mode de réalisation, c'est la partie solide qui comprend la chlorure de calcium.

La teneur du chlorure de calcium dans la partie solide peut par exemple être comprise entre 0,1 et 10% en poids de l'ensemble des constituants de la partie solide, et de préférence entre 0,9 et 7,5%.

La partie liquide peut comprendre l'agent réducteur d'eau.

La proportion de l'agent réducteur d'eau dans cette partie liquide est par exemple comprise entre 0,1 et 10% en poids par rapport au volume total de la partie liquide, avantageusement entre 1 et 5% en poids et de préférence entre 2 et 4%.

Dans un autre mode réalisation, c'est la partie solide qui comprend l'agent réducteur d'eau.

La teneur de l'agent réducteur d'eau dans la partie solide est par exemple comprise entre 0,01 et 3% en poids de l'ensemble des constituants de la partie solide, avantageusement entre 0,15 et 1,25% et de préférence entre 0,38 et 0,84%.

Les agents réducteurs d'eau du type plastifiant, tels que ceux mentionnés ci-dessus, peuvent être utilisés dans la partie liquide ou la partie solide.

Quelles que soient les applications envisagées pour les préparations suivant l'invention, on peut prévoir que le rapport volume sur masse entre la partie liquide et la partie solide est compris entre 0,1 et 0,3, avantageusement entre 0,15 et 0,25, et de préférence entre 0,17 et 0,23.

Cette variation de rapport peut notamment être assurée par le choix de l'agent réducteur d'eau.

Dans une version avantageuse de l'invention, les composants de la partie solide sont micronisés.

Préférentiellement, et notamment pour la restauration dentaire, au moins 90% des particules de chacun des constituants de la partie solide a une granulométrie inférieure à 10 µm.

L'invention concerne également un procédé de réalisation de la préparation pour réaliser un matériau de restauration de substance minéralisée, notamment dans le domaine dentaire, à partir de la préparation selon l'invention.

Suivant l'invention, on mélange la partie solide et la partie liquide en utilisant tout moyen transmettant au dit mélange une haute énergie.

Après un mélange homogène des deux parties liquide et solide, on obtient un produit de teinte blanchâtre qui peut alors être mis en oeuvre facilement.

Le matériau obtenu selon l'invention, qui ne comporte bien évidemment pas de mercure, est un produit minéral à plus de 95%, ce qui lui assure une bio-compatibilité excellente.

L'invention concerne également l'utilisation de la préparation selon l'invention pour obtenir un matériau de restauration des dents, un ciment de scellement apical, un substitut dentino-cémentaire, un matériau de fond de cavité et un matériau de comblement osseux de la mâchoire.

La préparation selon l'invention peut particulièrement être utilisée dans le domaine dentaire suivant, entre autres, les exemples de réalisation donnés ci-après qui permettent d'obtenir toutes les caractéristiques voulues évoquées précédemment.

Le matériau selon l'invention est notamment préconisé pour la restauration des dents, plus particulièrement mais non limitativement, des dents postérieures, molaires et prémolaires.

Il peut également être utilisé comme ciment de scellement apical, par voie canalaire ou par voie chirurgicale dite "rétrograde", ou encore comme substitut dentino-cémentaire, dans le cas de perforations canalaires ou du plancher pulpaire d'origine iatrogène ou pathologique, mais aussi comme fond de cavité avec ou sans exposition pulpaire.

Le matériau selon l'invention peut en outre être employé pour le comblement osseux de la mâchoire.

De par sa couleur blanchâtre, le matériau dentaire répond de manière tout à fait satisfaisante à l'impératif esthétique.

Il est également tout à fait envisageable de prévoir que la préparation, dans sa partie solide et/ou liquide, comprenne en outre un colorant pour augmenter la palette des couleurs possibles.

Ainsi, il devient possible de préparer un matériau dentaire dont la teinte finale s'adaptera parfaitement à la teinte des dents du patient.

D'autres propriétés et avantages de l'invention résulteront de la description qui va suivre, donnée à titre d'exemple non limitatif et faite en référence aux figures annexées dans lesquelles :
- la figure 1 est une reproduction d'une image observée au moyen d'un microscope électronique à balayage et réalisée sur la section polie d'une cavité de prémolaire remplie de ciment préparé à partir de la préparation selon l'invention ; et
- la figure 2 est une reproduction agrandie de la zone repérée par un cadre blanc sur la figure 1.

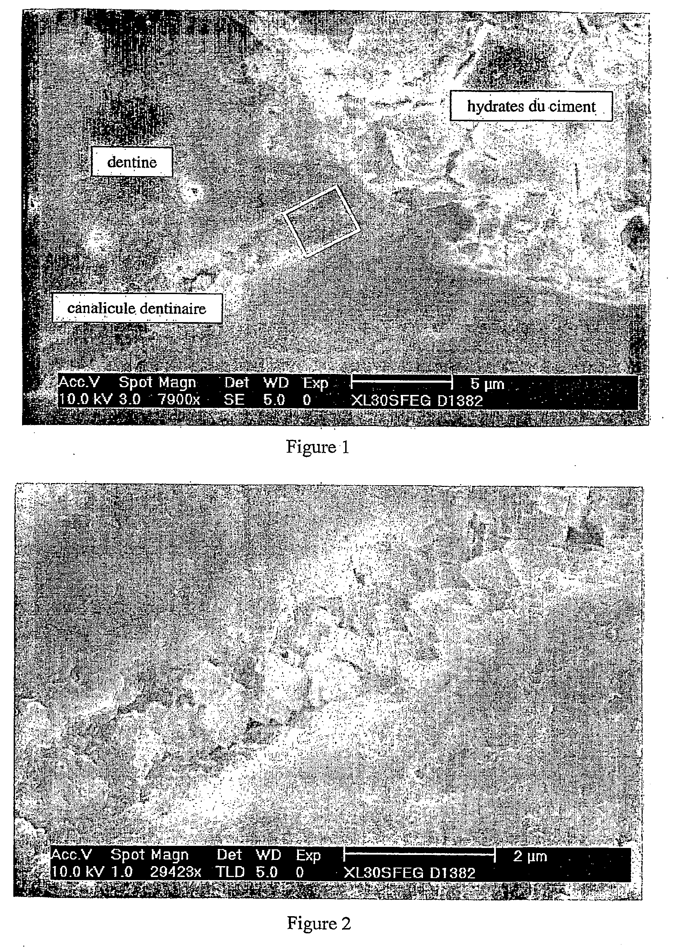

Le matériau suivant l'invention utilisé comme ciment dentaire pour restaurer la prémolaire, dont une partie est représentée sur la figure 1, a été préparé à partir de la préparation suivante, dans un rapport massique partie liquide/partie solide égal à 0,21 :
Partie solide (pour 100 g) :
   - Ca₃SiO₅ 85 g
   - CaCO₃ 15 g
Partie liquide (pour 100 ml) :
   - CaCl₂, 2H₂O 14,7 g
   - agent réducteur d'eau 3 g
   - eau q.s.p. 100 ml

Les matières premières pulvérulentes de la partie solide utilisées sont micronisées au moins pour cette application dentaire. De préférence et en général, leur granulométrie est inférieure à 10 microns.

Dans cet exemple, les caractéristiques granulométriques des différents constituants sont les suivantes :
- pour Ca₃SiO₅: d₁₀ = 0,81 µm
   d₅₀ = 3,16 µm
   d₉₀ = 7,51 µm,
      dₓ représentant la taille maximale atteinte par x% des particules du composé considéré, en l'espèce pour Ca₃SiO₅.
- pour CaCO₃, le diamètre moyen des particules est compris entre 50 et 100 nm.

Suivant un autre exemple de réalisation, la préparation suivant l'invention peut comporter de l'oxyde de zirconium ZᵣO₂.

Une telle préparation peut également constituer un ciment dentaire, en particulier dans les applications de scellement apical, par voie canalaire ou par voie chirurgicale dite "rétrograde", pour lesquelles on doit disposer d'un matériau ayant une bonne radio-opacité.

Un tel ciment est préparé à partir de la préparation suivante :
Partie solide (pour 100 g):
   - Ca₃SiO₅ 80,75 g
   - CaCO₃ 14,25 g
   - ZᵣO₂ 5,00 g
Partie liquide (pour 100 ml) :
   - CaCl₂, 2H₂O 14,7 g
   - agent réducteur d'eau 3 g
      .eau q.s.p. 100 ml,

le rapport massique partie liquide/partie solide étant dans le cas présent égal à 0,18.

Dans ce second exemple, les caractéristiques granulométriques de ZᵣO₂ sont les suivantes :
d₁₀ = 0,28 µm
dso = 0,71 µm
d₉₀ = 1,53 µm,
   dₓ étant défini comme précédemment et les caractéristiques granulométriques des deux autres constituants Ca₃SiO₅ et CaCO₃ étant identiques à celles de l'exemple précédent.

L'agent réducteur d'eau utilisé dans ces deux exemples de réalisation était un plastifiant de nouvelle génération, dit "superplastifiant", à base de polycarboxylate modifié commercialisé par la Société Chryso sous le nom commercial "Chrysofluid Premia".

Le praticien réalise le mélange des parties solide et liquide extemporanément puis procède à la mise en place du matériau dentaire obtenu, par exemple avec un porte-amalgame, pour les travaux dentaires envisagés.

Dans le cas présent, après mélange de l'une ou l'autre des préparations exemplifiées ci-dessus, le ciment a été introduit dans une cavité de prémolaire, au moyen de l'outillage conventionnel du praticien.

Dans les exemples ci-dessus, les mélanges des préparations considérées ont été réalisés par un moyen de mélange automatique transmettant aux préparations une haute énergie de façon à obtenir une pâte homogène qui peut alors ensuite être mise en place à l'aide d'un porte-amalgame.

Le temps de prise du matériau dans l'application dentaire selon l'invention est compatible avec la durée d'intervention du praticien et est obtenu sans intervention ni rajout de substance : cette prise se fait en effet en l'absence de tout additif du type monomères photo- ou chémo-polymérisables et sans recours à l'application d'ultrasons comme cela est le cas avec les résines actuelles.

Dans des conditions d'utilisation à 100% d'humidité et à 37° Celsius, on obtient alors un matériau dentaire ayant toutes les caractéristiques souhaitées telles que :
- pas de rétraction dans le temps avec une stabilité dimensionnelle parfaite,
- une résistance à la compression en 24 heures de 100 à 200 Mpa,
- une bonne adhésion au tissu dentaire comme illustrée sur les figures jointes,
- une esthétique acceptable,
- une non solubilité après la prise,
- une isolation électrique et thermique,
- une dureté compatible avec la mastication,
- une manipulation clinique de la préparation, et donc du matériau obtenu après mélange de cette préparation, à la fois simple et pratique.

Sur la figure 1, on repère la dentine au niveau de son interface formée avec la cavité de la prémolaire.

Cette cavité est comblée par le matériau, ou ciment dentaire, selon l'invention. Au niveau de la dentine, apparaît un canalicule dentinaire, dont une partie est repérée par le cadre blanc.

Sur la figure 2, l'agrandissement du cadre montre de manière détaillée l'intérieur du canalicule dentinaire. On constate que cet intérieur est caractérisé par la présence d'une grande quantité de cristaux.

Une analyse de ces cristaux a montré que ceux-ci comportaient du silicium, qui provient nécessairement du ciment dentaire introduit dans la cavité de la prémolaire.

Ce constat signifie que le matériau dentaire ainsi mis en oeuvre comble non seulement de façon remarquable toute la cavité de la dent, mais également qu'il pénètre à l'intérieur des canalicules dentinaires parcourant la dentine.

Ainsi, en créant des liaisons physico-chimiques avec les tissus minéralisés, et en particulier avec la dentine, le ciment dentaire permet d'obtenir une bonne étanchéité marginale.

Cette bonne étanchéité marginale est renforcée par le fait que le ciment dentaire conserve sa stabilité dimensionnelle : aucun phénomène de contraction linéaire n'est en effet observé.

En outre, ce ciment dentaire est doté des propriétés mécaniques requises pour un matériau d'obturation des dents, notamment des dents postérieures, et laisse augurer d'une durabilité supérieure à celle des résines composites.

Par ailleurs, le matériau obtenu à partir de la préparation selon l'invention présente une absence de cytotoxicité particulièrement intéressante.

L'évaluation de la cytotoxicité, et donc de l'activité toxique éventuelle du ciment contre certaines cellules conduisant à leur destruction, a été conduite selon le protocole AFNOR, norme S 99-505-5, NF EN, ISO 10 993-5 de décembre 1999.

Le taux de mortalité cellulaire observé à trois instants, respectivement 3 heures, 1 puis 7 jours après la prise du ciment, a dans les trois cas été inférieur à 10%, ce qui correspond à une toxicité nulle selon cette norme AFNOR.

Bien que la présente description ait été plus spécifiquement illustrée par une préparation pour reconstitution dentaire, elle concerne de manière générale un matériau de reconstitution de substance minéralisée, sans que ceci ne vienne, d'une quelconque manière, limiter la portée de l'invention.

Un tel matériau peut ainsi être utilisé comme matériau de scellement apical, par voie canalaire ou par voie chirurgicale dite "rétrograde", comme substitut dentino-cémentaire, dans le cas de perforations canalaires ou du plancher pulpaire d'origine iatrogène ou pathologique, comme fond de cavité avec ou sans exposition pulpaire, ou encore comme matériau de comblement osseux de la mâchoire.

## Revendications

1. Préparation pour réaliser un matériau de restauration de substance minéralisée, **caractérisée en ce qu'**elle comprend :
- une partie liquide aqueuse,
- une partie solide comprenant entre 70% et 99% 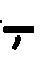 en poids de silicate dicalcique et/ou de silicate tricalcique, et entre 1 et 30% en poids de carbonate de calcium CaCO₃, ces pourcentages pondéraux étant donnés en référence à l'ensemble des constituants de la partie solide,
- du chlorure de calcium CaCl₂ et un agent réducteur d'eau, contenus chacun dans au moins l'une des dites parties, la partie solide et la partie liquide étant destinées à être mélangées pour obtenir ledit matériau.

2. Préparation selon la revendication 1,
**caractérisée en ce que** la partie solide comprend de l'oxyde de zirconium ZrO₂, par exemple entre 0 et 15% en poids de l'ensemble des constituants de la partie solide.

3. Préparation selon l'une quelconque des revendications 1 à 2, **caractérisée en ce que** la partie liquide comprenant le CaCl₂, par exemple avec une teneur comprise entre 1 et 35% en poids par rapport au volume total de cette partie liquide, et de préférence entre 9 et 25%.

4. Préparation selon l'une quelconque des revendications 1 à 2, **caractérisée en ce que** la partie solide comprend le CaCl₂, par exemple avec une teneur comprise entre 0,1 et 10% en poids de l'ensemble des constituants de la partie solide, et de préférence entre 0,9 et 7,5%.

5. Préparation selon l'une quelconque des revendications 1 à 4, **caractérisée en ce que** la partie liquide comprend l'agent réducteur d'eau, par exemple dans une proportion comprise entre 0,1 et 10% en poids par rapport au volume total de la partie liquide, avantageusement entre 1 et 5% et de préférence entre 2 et 4%.

6. Préparation selon l'une quelconque des revendications 1 à 4, **caractérisée en ce que** la partie solide comprend l'agent réducteur d'eau, par exemple dans une proportion comprise entre 0,01 et 3% en poids de l'ensemble des constituants de la partie solide, avantageusement entre 0,15 et 1,25% et de préférence entre 0,38 et 0,84%.

7. Préparation selon l'une quelconque des revendications 1 à 6, **caractérisée en ce que** l'agent réducteur d'eau est un plastifiant, par exemple du polynaphtalènesulfonate (PNS) ou un plastifiant à base de polycarboxylate modifié.

8. Préparation selon l'une quelconque des revendications 1 à 7, **caractérisée en ce que** le rapport volume sur masse entre la partie liquide et la partie solide est compris entre 0,1 et 0,3, avantageusement entre 0,15 et 0,25, et de préférence entre 0,17 et 0,23.

9. Préparation selon l'une quelconque des revendications 1 à 8, notamment pour la restauration dentaire, **caractérisée en ce qu'**au moins 90% des particules de chacun des constituants de la partie solide a une granulométrie inférieure à 10 µm.

10. Procédé pour réaliser un matériau de restauration de substance minéralisée, notamment dans le domaine dentaire, à partir de la préparation suivant l'une quelconque des revendications 1 à 9, **caractérisée en ce que** l'on effectue un mélange homogène des parties solide et liquide.

11. Utilisation de la préparation suivant l'une quelconque des revendications 1 à 9 pour obtenir un matériau de restauration des dents, un ciment de scellement apical, un substitut dentino-cémentaire, un matériau de fond de cavité et un matériau de comblement osseux de la mâchoire.

## Claims

1. A preparation for producing a material for restoring mineralised substance, **characterised in that** it comprises:
- an aqueous liquid portion,
- a solid portion comprising between 70% and 99% by weight of dicalcium silicate and/or tricalcium silicate and between 1 and 30% by weight of calcium carbonate CaCO₃, these percentages by weight being stated with reference to all the constituents of the solid portion,
- calcium chloride CaCl₂ and a water reducing agent, each contained in at least one of said portions, the solid portion and the liquid portion being intended to be mixed in order to obtain said material.

2. A preparation according to claim 1, **characterised in that** the solid portion comprises zirconium oxide ZrO₂, for example between 0 and 15% by weight of all the constituents of the solid portion.

3. A preparation according to either one of claims 1 and 2, **characterised in that** the liquid portion comprises the CaCl₂, for example with a content of between 1 and 35% by weight relative to the total volume of said liquid portion, and preferably between 9 and 25%.

4. A preparation according to either one of claims 1 and 2, **characterised in that** the solid portion comprises the CaCl₂, for example with a content of between 0.1 and 10% by weight relative to all the constituents of the solid portion, and preferably between 0.9 and 7.5%.

5. A preparation according to any one of claims 1 to 4, **characterised in that** the liquid portion comprises the water reducing agent, for example in a proportion of between 0.1 and 10% by weight relative to the total volume of the liquid portion, advantageously between 1 and 5% and preferably between 2 and 4%.

6. A preparation according to any one of claims 1 to 4, **characterised in that** the solid portion comprises the water reducing agent, for example in a proportion of between 0.01 and 3% by weight relative to all the constituents of the solid portion, advantageously between 0.15 and 1.25% and preferably between 0.38 and 0.84%.

7. A preparation according to any one of claims 1 to 6, **characterised in that** the water reducing agent is a plasticiser, for example polynaphthalene sulfonate (PNS) or a plasticiser based on modified polycarboxylate.

8. A preparation according to any one of claims 1 to 7, **characterised in that** the volume to mass ratio between the liquid portion and the solid portion is between 0.1 and 0.3, advantageously between 0.15 and 0.25, and preferably between 0.17 and 0.23.

9. A preparation according to any one of claims 1 to 8, in particular for dental restoration, **characterised in that** at least 90% of the particles of each of the constituents of the solid portion has a particle size of less than 10 µm.

10. A process for producing a material for restoring mineralised substance, in particular in the dental field, from the preparation according to any one of claims 1 to 9, **characterised in that** the solid and liquid portions are mixed homogeneously.

11. Use of the preparation according to any one of claims 1 to 9 to obtain a dental restorative material, an apical sealing cement, a dentino-cemental substitute, a cavity base material and a jaw bone filling material.
Translator's comment
Claim 3: "comprenant" ("comprising") has been assumed to be in error for "comprend" ("comprises") (see claim 4) and translated accordingly.

## Patentansprüche

1. Zusammensetzung zur Herstellung von einem Restaurationsmaterial einer mineralischen Substanz, **dadurch gekennzeichnet, dass** sie enthält:
- einen wässrigen Flüssiganteil,
- einen Feststoffanteil mit zwischen 70 Gew.-% und 99 Gew.-% Dicalciumsilikat und/oder Tricalciumsilikat und zwischen 1 und 30 Gew.-% Calciumcarbonat CaCO₃, wobei diese Gewichtsprozentwerte im Verhältnis auf die gesamten Bestandteile des Feststoffanteils angegeben sind,
- Calciumchlorid CaCl₂ und ein Wasserreduktionsmittel, die jeweils in zumindest einem der genannten Anteile enthalten sind, wobei der Feststoffanteil und der Flüssiganteil dazu bestimmt sind, vermischt zu werden, um das genannte Material zu erhalten.

2. Zusammensetzung nach Anspruch 1, **dadurch gekennzeichnet, dass** der Feststoffanteil Zirkoniumoxid ZrO₂, beispielsweise zwischen 0 und 15 Gew.-% der gesamten Bestandteile des Feststoffanteils, enthält.

3. Zusammensetzung nach einem der Ansprüche 1 und 2, **dadurch gekennzeichnet, dass** der Flüssiganteil CaCl₂ enthält, beispielsweise mit einem Gehalt von zwischen 1 und 35 Gew.-% im Verhältnis zum Gesamtvolumen dieses Flüssiganteils und vorzugsweise zwischen 9 und 25 %.

4. Zusammensetzung nach einem der Ansprüche 1 und 2, **dadurch gekennzeichnet, dass** der Feststoffanteil CaCl₂ enthält, beispielsweise mit einem Gehalt von zwischen 0,1 und 10 Gew.-% der gesamten Bestandteile des Feststoffanteils und vorzugsweise zwischen 0,9 und 7,5 %.

5. Zusammensetzung nach einem der Ansprüche 1 bis 4, **dadurch gekennzeichnet, dass** der Flüssiganteil ein Wasserreduktionsmittel enthält, beispielsweise in einer Proportion von zwischen 0,1 und 10 Gew.-% im Verhältnis zum Gesamtvolumen des Flüssiganteils, vorteilhaft zwischen 1 und 5 % und bevorzugt zwischen 2 und 4 %.

6. Zusammensetzung nach einem der Ansprüche 1 bis 4, **dadurch gekennzeichnet, dass** der Feststoffanteil ein Wasserreduktionsmittel enthält, beispielsweise in einer Proportion von zwischen 0,01 und 3 Gew.-% der gesamten Bestandteile des Feststoffanteils, vorteilhaft zwischen 0,15 und 1,25 % und bevorzugt zwischen 0,38 und 0,84 %.

7. Zusammensetzung nach einem der Ansprüche 1 bis 6, **dadurch gekennzeichnet, dass** das Wasserreduktionsmittel ein Weichmacher ist, beispielsweise Polynaphtalensulfonat (PNS), oder ein Weichmacher auf Basis von modifiziertem Polycarboxylat.

8. Zusammensetzung nach einem der Ansprüche 1 bis 7, **dadurch gekennzeichnet, dass** das Volumen-Masse-Verhältnis zwischen dem Flüssiganteil und dem Feststoffanteil zwischen 0,1 und 0,3, vorteilhaft zwischen 0,15 und 0,25 und bevorzugt zwischen 0,17 und 0,23 liegt.

9. Zusammensetzung nach einem der Ansprüche 1 bis 8, insbesondere zur Zahnrestauration, **dadurch gekennzeichnet, dass** zumindest 90 % der Partikel eines jeden Bestandteils des Feststoffanteils eine Korngröße kleiner als 10 µm aufweist.

10. Verfahren zum Herstellen von einem Restaurationsmaterial einer mineralischen Substanz, insbesondere im zahnmedizinischen Bereich, ausgehend von einer Zusammensetzung nach einem der Ansprüche 1 bis 9, **dadurch gekennzeichnet, dass** eine homogene Mischung des Feststoff- und des Flüssiganteils erfolgt.

11. Verwendung der Zusammensetzung nach einem der Ansprüche 1 bis 9 zum Erhalt von Zahnrestaurationsmaterial, apikalem Befestigungszement, Dentin-Zahnzement-Ersatz, Kavitätenmaterial und Kieferknochenfüllmaterial.
